# EUROPÄISCHE PATENTSCHRIFT

(11) **EP 2 032 520 B1**
(45) Veröffentlichungstag und Bekanntmachung des Hinweises auf die Patenterteilung: **21.11.2018**
(21) Anmeldenummer: 07726889.4
(22) Anmeldetag: 14.03.2007
(51) Int. Cl.: C07C 57/07

(54) **VERFAHREN ZUR AUFREINIGUNG VON POLYMERISIERBAREN VERBINDUNGEN**
METHOD FOR PURIFYING POLYMERIZABLE COMPOUNDS
PROCÉDÉ DE PURIFICATION DE COMPOSÉS POLYMÉRISABLES

(30) Priorität: 23.06.2006 DE 102006029319
(43) Veröffentlichungstag der Anmeldung: 11.03.2009
(73) Patentinhaber: Evonik Röhm GmbH, 64293 Darmstadt (DE)
(72) Erfinder: BRÖLL, Dirk, 63225 Langen (DE); SIEGERT, Hermann, 64342 Seeheim-Jugenheim (DE)
(86) Internationale Anmeldenummer: PCT/EP2007/052397
(87) Internationale Veröffentlichungsnummer: WO 2007/147651

(56) Entgegenhaltungen:
- EP-A- 0 188 775
- DE-A1- 10 256 147
- US-A- 5 154 800
- US-A1- 2005 077 240

## Beschreibung

Die Erfindung beschreibt ein Verfahren zur destillativen Aufreinigung von polymerisierbaren Verbindungen und die Verwendung eines Siedeöls zur destillativen Aufreinigung von polymerisierbaren Verbindungen.

DE 102 56 147 A1 beschreibt ein Verfahren zur rektifikativen Auftrennung von (Meth)acrylmonomere enthaltenden Flüssigkeiten in einer Rektifikationskolonne, bei dem aus der Rektifikationskolonne ein Stoffstrom entnommen und als mit molekularem Sauerstoff angereicherte Flüssigphase in die Rektifikationskolonne zurückgeführt wird.
In DE-A-2136396 wird ein Verfahren zur Gewinnung wasserfreier Acrylsäure durch Gegenstromwäsche der Reaktionsgase in einer Absorberkolonne mit einem hochsiedenden inerten extrem hydrophoben Lösungsmittel beschrieben. Geeignete Lösungsmittel sind Kohlenwasserstoffe der Mittelölfraktion, Wärmeträgeröle mit Siedepunkten oberhalb 170 °C (bei Normaldruck) oder Diphenylether, Diphenyl und/oder deren Gemische. Das Lösungsmittel wird über den Kopf der Kolonne zugeführt. Für die Absorption wird eine möglichst niedrige Temperatur von 30-80°C bei Normaldruck eingestellt.

Aus der EP-A-188775 ist ein Verfahren zur Gewinnung wasserfreier Methacrylsäure bekannt, bei dem die anfallenden Reaktionsgase insbesondere durch Gegenstromwäsche in einer Absorberkolonne mit einem inerten, hochsiedenden hydrophoben organischen Lösungsmittel gewaschen werden. Das Lösungsmittel wie Diphenyl, Diphenyläther, Dibenzofuran und/oder deren Gemische wird über den Kopf der Kolonne zugegeben. Die Absorptionstemperatur beträgt 40-120 °C bei Normaldruck.

Nachteilig bei den vorgenannten Verfahren ist, dass zur Gewinnung von wasserfreier Acrylsäure bzw. Methacrylsäure weitere Desorptions- und Destillationsschritte nachfolgen müssen, um das Zielprodukt von dem verwendeten Lösungsmittel wieder abzutrennen.

Aufgabe der vorliegenden Erfindung ist es, ein Verfahren zur destillativen Aufreinigung von polymerisierbaren Verbindungen bereitzustellen, bei dem die als Hilfsmittel verwendeten Substanzen ohne weitere Aufreinigung in die Anlage zurückgeführt werden können und maximal 10% des Hilfsmittels bezogen auf das Zielprodukt ausgeschleust werden. Darüber hinaus soll das Verfahren vor allem gewährleisten, dass eine Polymerisation des Zielprodukts vermieden wird.
Die Aufgabe wird dadurch gelöst, dass die destillative Aufreinigung der polymerisierbaren Verbindung in Gegenwart einer hochsiedenden, inerten thermisch langzeitstabilen Substanz durchgeführt wird, wobei diese als Siedeöl bezeichnete Substanz im Sumpf einer Rektifikationskolonne zugegen ist. Dadurch werden lange Verweilzeiten des polymerisationsanfälligen Zielproduktes im Sumpf ausgeschlossen, da die Konzentration der polymerisationsanfälligen Verbindung durch den Wärmeaustausch mit den Siedeöldämpfen in Richtung Sumpf und somit in Richtung zunehmender Temperatur stark abnimmt, wodurch die Polymerisationsgefahr weitgehend gebannt ist.

Gegenstand der Erfindung ist daher ein Verfahren zur destillativen Aufreinigung von polymerisierbaren Verbindungen unter Verwendung einer hoch siedenden, inerten thermisch langzeitstabilen Substanz als Siedeöl, dadurch gekennzeichnet, dass sich das Siedeöl im Sumpf einer Rektifikationskolonne befindet und maximal 10% des Hilfsmittels bezogen auf das Zielprodukt ausgeschleust werden, wobei der Siedepunkt des Siedeöls höher ist als der Siedepunkt des reinen Zielprodukts und als hoch siedende, inerte thermisch langzeitstabile Substanz höherkettige unverzweigte Paraffine mit 12-20-Kohlenstoffatomen, aromatische Verbindungen wie Diphyl, alkylsubstituierte Phenole oder Naphthalinverbindungen, Sulfolan oder Mischungen aus diesen verwendet werden und wobei das reine Zielprodukt an einem Seitenabzug unterhalb des mittleren Bereichs der Kolonne ausgetragen wird.

Für das erfindungsgemäße Verfahren wird als Siedeöl eine hochsiedende, inerte thermisch langzeitstabile Substanz mit einem Siedepunkt höher als der Siedepunkt des reinen Zielprodukts verwendet, um dessen destillative Abtrennung zu gewährleisten. Der Siedepunkt des Siedöls sollte aber auch nicht zu hoch sein, um die thermische Belastung der reinen polymerisierbaren Verbindung zu reduzieren.
Im Allgemeinen liegt die Siedetemperatur des Siedeöls bei Normaldruck (1013 mbar) bei 150 bis 400°C, insbesondere bei 200 bis 300 °C.

Geeignete Siedeöle sind u.a. höherkettige unverzweigte Paraffine mit 12-20-Kohlenstoffatomen, aromatische Verbindungen wie Diphyl (eutektische Mischung aus 75% Biphenyloxid und 25% Biphenyl), alkylsubstituierte Phenole oder Naphthalinverbindungen, Sulfolan (Tetrahydro-thiophen-1,1-dioxid), oder Mischungen aus diesen.

Geeignete Beispiele sind die nachfolgend aufgeführten Siedeöle:

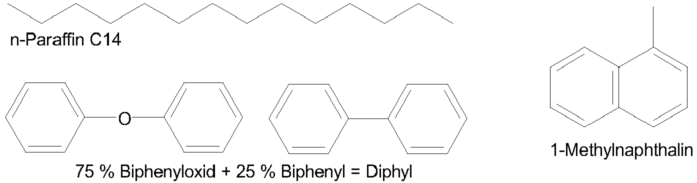

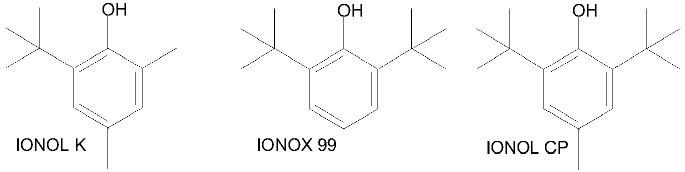

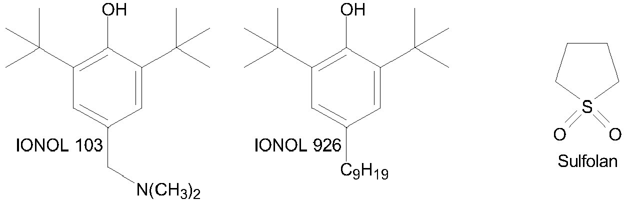

Besonders bevorzugt werden 2,6-di-tert-Butyl-para-cresol, 2,6-di-tert-Butyl-phenol, Sulfolan, Diphyl oder Mischungen aus diesen eingesetzt, ganz besonders bevorzugt Sulfolan.

Für das erfindungsgemäße Verfahren kann jede Rektifikationskolonne verwendet werden, die vorzugsweise 5 bis 50 Trennstufen besitzt. Als Anzahl der Trennstufen wird in der vorliegenden Erfindung die Anzahl der Böden bei einer Bodenkolonne multipliziert mit dem Bodenwirkungsgrad oder die Anzahl der theoretischen Trennstufen im Fall einer Packungskolonne oder eine Kolonne mit Füllkörpern bezeichnet.

Beispiele für eine Rektifikationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine Rektifikationskolonne mit Füllkörpern solche wie Raschig-Ringe, Lessing-Ringe, Pall-Ringe, Berl-Sättel, Intalox Sättel und für eine Rektifikationskolonne mit Packungen solche wie vom Typ Mellapak (Sulzer), Rombopak (Kühni), Montz-Pak (Montz) und Packungen mit Katalysatortaschen, beispielsweise Katapak (Sulzer).

Eine Rektifikationskolonne mit Kombinationen aus Bereichen von Böden, aus Bereichen von Füllkörpern und/oder aus Bereichen von Packungen kann ebenso verwendet werden.

Bevorzugt wird eine Rektifikationskolonne mit Füllkörpern und/oder Packungen eingesetzt. Die Rektifikationskolonne kann aus jedem hierfür geeigneten Material hergestellt werden. Hierzu gehören u.a. Edelstahl sowie inerte Materialien.

Vorzugsweise wird die Rektifikationskolonne im Vakuum bei einem Absolutdruck von 1 bis 500 mbar, vorzugsweise bei einem Absolutdruck von 1 bis 100 mbar, betrieben. Die Temperatur im Sumpf der Rektifikationskolonne ergibt sich durch das verwendete Siedeöl und den herrschenden Systemdruck.

Unter polymerisierbaren Verbindungen sind im allgemeinen Monomere mit mindestens einer reaktionsfähigen Doppelbindung oder anderen reaktiven funktionellen Gruppen zu verstehen. Zu ihnen zählen u.a. Verbindungen mit Kohlenstoff-Kohlenstoff-Mehrfachbindungen (Olefine, Alkine, Vinyl-, (Meth)acryl-Verbindungen), cyclische Ether, Ester oder Amide (Oxirane, Lactone, Lactame), ungesättigte cyclische Kohlenwasserstoffe sowie solche mit Isocycanat- oder H-aciden Amino-, Hydroxy- oder Carboxy-Gruppen. Geeignete polymerisierbare Verbindungen sind dem Fachmann aus der Literatur wie zum Beispiel aus J. Brandrup, E. H. Immergut und E. A. Grulke, Polymer Handbook, 4. Aufl., Hoboken, John Wiley and Sons, 1999, Seiten III-1 bis III-41. bekannt, worauf explizit Bezug genommen wird.

Die Zufuhr der aufzureinigenden polymerisierbaren Verbindung erfolgt vorzugsweise oberhalb des mittleren Bereiches der Kolonne. Niedrig siedende Verunreinigungen werden am Kopf der Kolonne abgezogen, hochsiedende Verunreinigungen werden aus dem Kolonnensumpf ausgeschleust. Das reine Zielprodukt wird an einem Seitenabzug unterhalb des mittleren Bereiches der Kolonne ausgetragen.

Die Kolonne kann noch mit anderen Apparaten verschaltet sein, wie beispielsweise weiteren Apparaten zur Stofftrennung und/oder einem Reaktor. Ein Reaktionsbereich kann auch innerhalb der Kolonne angeordnet sein. Die Kolonne kann auch in mehrere Trennsegmente unterteilt sein, die verschiedene Aufgaben erfüllen.

Um unerwünschte Polymerisationen der aufzureinigenden polymerisierbaren Verbindung zu vermeiden wird gegebenenfalls ein Polymerisationsinhibitor zugefügt. Zu den bevorzugt einsetzbaren Polymerisationsinhibitoren gehören unter anderem Octadecyl-3-(3,5-di-tert-butyl-4-hydroxyphenyl)-propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierte Phenylendiamine wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon oder Gemische aus zwei oder mehreren dieser Stabilisatoren.
Die Zudosierung des Inhibitors erfolgt vorzugsweise am Kopf der Kolonne. Aus dem Kolonnensumpf können Hochsieder wie zugesetzte Inhibitoren durch übliche Methoden ausgeschleust werden, beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Gegenstand der Erfindung ist ferner die Verwendung der obengenannten hochsiedenden, inerten thermisch langzeitstabilen Substanz als Siedeöl im Sumpf einer Rektifikationskolonne zur destillativen Aufreinigung von polymerisierbaren Verbindungen.
Das erfindungsgemäße Verfahren ermöglicht es, die polymerisierbare Verbindung ohne Verluste durch unerwünschte Polymerisation in hoher Reinheit durch einfache Abtrennung zu erhalten, wobei das verwendete Siedeöl ohne weitere Aufreinigung in die Anlage zurückgeführt werden kann.

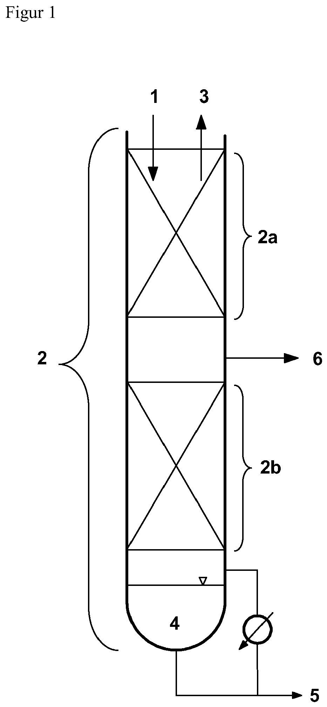

Eine Ausführungsform des erfindungsgemäßen Verfahrens ist schematisch in Figur 1 dargestellt.

Das aufzureinigende Monomer = Rohmonomer (1) gelangt in den unteren Teil einer Rektifikationskolonne (2). Hier findet im Trennbereich (2a) die Abtrennung von Komponenten, die leichter (3) als das aufzureinigende Monomer sieden statt. Im Trennbereich (2b) der Kolonne wird das Monomer von dem im Sumpf befindlichen Siedeöl (4) sowie von Komponenten, die höher als das aufzureinigende Monomer sieden getrennt. Im Sumpf befindliche Hochsieder können durch übliche Methoden ausgeschleust werden (5), beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust. Das hochreine Monomer (6) wird zwischen Trennbereich (2a) und (2b) bevorzugt gasförmig abgezogen.

Die nachfolgenden Beispiele veranschaulichen das erfindungsgemäße Verfahren ohne dieses darauf zu beschränken.

### Beispiel 1: Aufreinigung von Methacrylsäureanhydrid

Die Aufreinigung von Methacrylsäureanhydrid wurde im unteren Teil einer Rektifikationskolonne gemäß Figur 1 durchgeführt.

Die Rektifikationskolonne hatte im Trennbereich (2a) zwölf und im Trennbereich (2b) acht Trennstufen. Diese Kolonne, hatte einen Innendurchmesser von 100 mm und war mit Packungen der Fa. Sulzer, Typ CY (Trennbereich 2a) und der Fa. Montz, Typ BSH 400 (Trennbereich 2b) bestückt. Der Druck im Kolonnensumpf betrug 35 mbar. Bei stationären Bedingungen stellte sich ein Temperaturprofil von 164 °C (Sumpf) bis 66 °C (oberes Ende des Trennbereiches 2a) ein. Der Austrag an Methacrylsäureanhydrid am Seitenabzug (zwischen Trennbereiches 2a und 2b) sowie die Heizdampfleistung des Sumpfverdampfers erfolgte temperaturgeregelt in den jeweiligen Bereichen.

Im Sumpf der Rektifikationskolonne wurden 6 kg Sulfolan als Siedeöl (4) eingesetzt. Als Verdampfer diente ein Fallfilmverdampfer.

Am Seitenstromabzug wurde Methacrylsäureanhydrid mit einer Reinheit von 99,7 % (GC-Analyse) entnommen.

### Beispiel 2: Aufreinigung von Acrylsäureanhydrid

Die Aufreinigung von Acrylsäureanhydrid wurde im selben unteren Teil einer Rektifikationskolonne gemäß Figur 1 wie in Beispiel 1 erläutert durchgeführt. Der Druck im Kolonnensumpf betrug 35 mbar. Bei stationären Bedingungen stellte sich ein Temperaturprofil von 164 °C (Sumpf) bis 54 °C (oberes Ende des Trennbereiches 2a) ein. Der Austrag an Acrylsäureanhydrid am Seitenabzug (zwischen Trennbereiches 2a und 2b) sowie die Heizdampfleistung des Sumpfverdampfers erfolgte temperaturgeregelt in den jeweiligen Bereichen.

Im Sumpf der Rektifikationskolonne wurden 6 kg Sulfolan als Siedeöl (4) eingesetzt. Als Verdampfer diente ein Fallfilmverdampfer.

Am Seitenstromabzug wurde Acrylsäureanhydrid mit einer Reinheit von 99,7 % (GC-Analyse) entnommen.

## Patentansprüche

1. Verfahren zur destillativen Aufreinigung von polymerisierbaren Verbindungen unter Verwendung einer hoch siedenden, inerten thermisch langzeitstabilen Substanz als Siedeöl, **dadurch gekennzeichnet, dass** sich das Siedeöl im Sumpf einer Rektifikationskolonne befindet und maximal 10% des Hilfsmittels bezogen auf das Zielprodukt ausgeschleust werden, wobei der Siedepunkt des Siedeöls höher ist als der Siedepunkt des reinen Zielprodukts
und als hoch siedende, inerte thermisch langzeitstabile Substanz höherkettige unverzweigte Paraffine mit 12-20-Kohlenstoffatomen, aromatische Verbindungen wie Diphyl, alkylsubstituierte Phenole oder Naphthalinverbindungen, Sulfolan oder Mischungen aus diesen verwendet werden und wobei das reine Zielprodukt an einem Seitenabzug unterhalb des mittleren Bereichs der Kolonne ausgetragen wird.

2. Verfahren gemäß Anspruch 1, **dadurch gekennzeichnet, dass** der Siedepunkt des Siedeöls bei 1013 mbar 150 bis 400 °C beträgt.

3. Verfahren gemäß Anspruch 1 oder 2, **dadurch gekennzeichnet, dass** der Siedepunkt des Siedeöls bei 1013 mbar 200 bis 300 °C beträgt.

4. Verfahren gemäß einem der Ansprüche 1 bis 3, **dadurch gekennzeichnet, dass** als hoch siedende, inerte thermisch langzeitstabile Substanz 2,6-di-tert-Butyl-para-cresol, 2,6-di-tert-Butyl-phenol, Sulfolan oder Diphyl oder Mischungen aus diesen verwendet wird.

5. Verfahren gemäß einem der Ansprüche 1 bis 4, **dadurch gekennzeichnet, dass** als hoch siedende, inerte thermisch langzeitstabile Substanz Sulfolan verwendet wird.

6. Verwendung einer hoch siedenden, inerten thermisch langzeitstabilen Substanz als Siedeöl im Sumpf einer Rektifikationskolonne, wobei maximal 10% des Hilfsmittels bezogen auf das Zielprodukt ausgeschleust werden, der Siedepunkt des Siedeöls höher ist als der Siedepunkt des reinen Zielprodukts und als hoch siedende, inerte thermisch langzeitstabile Substanz höherkettige unverzweigte Paraffine mit 12-20-Kohlenstoffatomen, aromatische Verbindungen wie Diphyl, alkylsubstituierte Phenole oder Naphthalinverbindungen, Sulfolan oder Mischungen aus diesen verwendet werden und wobei das reine Zielprodukt an einem Seitenabzug unterhalb des mittleren Bereichs der Kolonne ausgetragen wird, zur destillativen Aufreinigung von polymerisierbaren Verbindungen.

## Claims

1. Process for distillatively purifying polymerizable compounds using a high-boiling, inert, thermally long-term-stable substance as a boiling oil, **characterized in that** the boiling oil is disposed in the bottom of a rectification column and not more than 10% of the assistant, based on the target product, is discharged, where the boiling point of the boiling oil is higher than the boiling point of the pure target product,
and the high-boiling, inert, thermally long-term-stable substances used are relatively long-chain unbranched paraffins having 12-20 carbon atoms, aromatic compounds such as Diphyl, alkyl-substituted phenols or naphthalene compounds, sulpholane or mixtures thereof and where the pure target product is discharged at a side draw below the middle region of the column.

2. Process according to Claim 1, **characterized in that** the boiling point of the boiling oil at 1013 mbar is 150 to 400°C.

3. Process according to Claim 1 or 2, **characterized in that** the boiling point of the boiling oil at 1013 mbar is 200 to 300°C.

4. Process according to one of Claims 1 to 3, **characterized in that** the high-boiling, inert, thermally long-term-stable substance used is 2,6-di-tert-butyl-para-cresol, 2,6-di-tert-butylphenol, sulpholane or Diphyl, or mixtures thereof.

5. Process according to one of Claims 1 to 4, **characterized in that** the high-boiling, inert, thermally long-term-stable substance used is sulpholane.

6. Use of a high-boiling, inert, thermally long-term-stable substance as a boiling oil in the bottom of a rectification column, where not more than 10% of the assistant, based on the target product, is discharged, the boiling point of the boiling oil is higher than the boiling point of the pure target product
and the high-boiling, inert, thermally long-term-stable substances used are relatively long-chain unbranched paraffins having 12-20 carbon atoms, aromatic compounds such as Diphyl, alkyl-substituted phenols or naphthalene compounds, sulpholane or mixtures thereof and where the pure target product is discharged at a side draw below the middle region of the column, for the distillative purification of polymerizable compound.

## Revendications

1. Procédé de purification par distillation de composés polymérisables utilisant une substance inerte de point d'ébullition élevé thermiquement stable à long terme en tant qu'huile d'ébullition, **caractérisé en ce que** l'huile d'ébullition se trouve dans le fond d'une colonne de rectification et au plus 10 % de l'agent auxiliaire par rapport au produit cible est évacué, le point d'ébullition de l'huile d'ébullition étant supérieur au point d'ébullition du produit cible pur,
et des paraffines non ramifiées à chaîne longue de 12 à 20 atomes de carbone, des composés aromatiques tels que le Diphyl, les phénols à substitution alkyle ou les composés de naphtaline, le sulfolane ou des mélanges de ceux-ci étant utilisés en tant que substance inerte de point d'ébullition élevé thermiquement stable à long terme, et le produit cible pur étant déchargé par une sortie latérale en dessous de la zone centrale de la colonne.

2. Procédé selon la revendication 1, **caractérisé en ce que** le point d'ébullition de l'huile d'ébullition à 1 013 mbar est de 150 à 400 °C.

3. Procédé selon la revendication 1 ou 2, **caractérisé en ce que** le point d'ébullition de l'huile d'ébullition à 1 013 mbar est de 200 à 300 °C.

4. Procédé selon l'une quelconque des revendications 1 à 3, **caractérisé en ce que** du 2,6-di-tert-butyl-para-crésol, du 2,6-di-tert-butyl-phénol, du sulfolane ou du Diphyl ou des mélanges de ceux-ci sont utilisés en tant que substance inerte de point d'ébullition élevé thermiquement stable à long terme.

5. Procédé selon l'une quelconque des revendications 1 à 4, **caractérisé en ce que** du sulfolane est utilisé en tant que substance inerte de point d'ébullition élevé thermiquement stable à long terme.

6. Utilisation d'une substance inerte de point d'ébullition élevé thermiquement stable à long terme en tant qu'huile d'ébullition dans le fond d'une colonne de rectification, au plus 10 % de l'agent auxiliaire par rapport au produit cible étant évacué, le point d'ébullition de l'huile d'ébullition étant supérieur au point d'ébullition du produit cible pur,
et des paraffines non ramifiées à chaîne longue de 12 à 20 atomes de carbone, des composés aromatiques tels que le Diphyl, les phénols à substitution alkyle ou les composés de naphtaline, le sulfolane ou des mélanges de ceux-ci étant utilisés en tant que substance inerte de point d'ébullition élevé thermiquement stable à long terme, et le produit cible pur étant déchargé par une sortie latérale en dessous de la zone centrale de la colonne,
pour la purification par distillation de composés polymérisables.
